(19) Europäisches Patentamt

European Patent Office

Office européen des brevets

(11) **EP 1 105 335 B1**

(12) **EUROPEAN PATENT SPECIFICATION**

(45) Date of publication and mention
of the grant of the patent:
**07.07.2004 Bulletin 2004/28**

(51) Int Cl.⁷: **B65H 59/18**, B65H 59/40,
B65H 59/16

(21) Application number: **99923181.4**

(22) Date of filing: **18.05.1999**

(86) International application number:
**PCT/US1999/010936**

(87) International publication number:
**WO 1999/059908 (25.11.1999 Gazette 1999/47)**

(54) **FULL-COMPENSATING TENSION CONTROLLER**

FADENSPANNUNGSREGELVORRICHTUNG MIT VOLLSTÄNDIGER KOMPENSATION

CONTROLEUR DE TENSION A COMPENSATION COMPLETE

(84) Designated Contracting States:
**CH DE IT LI**

(30) Priority: **20.05.1998 US 86105 P**

(43) Date of publication of application:
**13.06.2001 Bulletin 2001/24**

(73) Proprietor: **Texkimp Limited**
**Northwich, Cheshire CW9 7NN (GB)**

(72) Inventor: **Niederer, Kurt W.**
**Charlotte, NC 28210-4829 (US)**

(74) Representative: **Pidgeon, Robert John et al**
**Appleyard Lees**
**15 Clare Road**
**Halifax West Yorkshire HX1 2HY (GB)**

(56) References cited:
| | |
|---|---|
| **DE-C- 180 006** | **FR-A- 1 501 879** |
| **GB-A- 983 286** | **GB-A- 1 101 480** |
| **US-A- 2 034 267** | **US-A- 2 586 037** |
| **US-A- 3 034 744** | **US-A- 3 113 746** |
| **US-A- 3 797 775** | **US-A- 4 526 329** |
| **US-A- 5 324 909** | **US-A- 5 454 151** |

**Description**

TECHNICAL FIELD AND BACKGROUND OF THE INVENTION

This application is based on provisional patent application Serial.No. 60/086,105, from which priority is claimed.

This invention relates to an apparatus and method for controlling the tension in moving yarns. More specifically, it compensates for varying tension over the time of a process and results in consistent yarn tension, which is often desirable for the next downstream process. As a further refinement on the principle of outputting a uniformly tensioned yarn at a single station is a mcthod and apparatus for remotely adjusting the tension of a group of tension devices simultaneously at any time during the process as well as an individual fine-tuning of the tension for each individual yarn is disclosed.

Numerous types of tension devices are known for controlling yarn tension. These include mostly devices which add constant tension to the traveling yarn and through this method reduce the percentage of the fluctuating tension. Most of those apply pressure directly to the traveling yarn, which in turn adds tension, based on the product of applied force times the friction coefficient. However, frictional forces directly applied to the yarn can damage the yarn itself. Another problem with this kind of tension device is that the yarn, which is pinched between two stationary members, can cause additional irregular tension, this is especially the case if the yarn is of uneven thickness. If for example a thick place in the traveling yarn passes this pinching place, the members are forced apart, causing a tension peak due to the mass of the stationary members, resisting the opening motion of the thick place in the yarn. Another problem with a frictional tension device is the variation of the friction coefficient of the yarn. This is especially true for unevenly waxed or oiled yarns.

More sophisticated yarn tensioning systems use complex and expensive electronic means to measure the yarn tension and electronically vary the applied tension with a close-loop feedback to achieve constant output tension.

FR-B-1501879 describes an apparatus intended to maintain constant yarn tension. Yarn from a yarn supply is redirected by a first pulley, and then by a second pulley. The second pulley carries an arm, one end of which is restrained by an adjustable spring. By this means changes in the downstream yarn tension may be compensated for.

US-B-5,454,151 describes an arrangement for adjusting the tension of a thread. The arrangement includes a driven drum whose effective circumferential speed is alterable in the same sense as the measured thread tension.

DE-B-180006 describes an arrangement for regulating yarn tension. The yarn is drawn from a yarn supply and is redirected over a first pulley, is then wound around a braking pulley, is then directed over a third pulley, which redirects it towards a yarn take-up machine. The third pulley is mounted at one end of a lever having a fulcrum. Changes in the downstream yarn tension act on the third pulley, and cause the lever to pivot. A brake shoe carried at the other end of the lever, and located so as to act on the rim of the braking pulley, is moved, thereby changing the braking force on the braking pulley, and altering the yarn tension.

The invention disclosed in this application employs a rotating yarn whorl around which the yarn is wrapped with sufficient wrapping angle to prevent slippage between the yarn and the yarn whorl during normal operation. Tension is applied to the yarn by braking the yarn whorl through means of mechanical frictional force, electrical eddy-current and others. The disclosed invention achieves constant output tension by reducing the applied tension by the same value as the amount of upstream tension of the yarn. Since the total downstream tension is the sum of the tension upstream of the tension device and the tension added by the tension device, the downstream tension in the disclosed invention is constant.

The invention works with the principle that the tension of the upstream is used as the means to change the applied tension of the tension controller. In a preferred method the tension of the upstream yarn strand is pulling the yarn whorl partially away from a tension generating brake and through this means reduces the added tension. The geometry of the braking force is chosen in such a manner as to reduce the set tension by exactly the same amount as the tension residing in the upstream yarn strand, hence achieving constant tension in the downstream yarn strand. It is naturally understood that if the incoming yarn tension exceeds the preset tension of the tensioning system, the yarn whorl is lifted completely from the brake shoe and the full upstream tension is transmitted downstream.

OBJECT AND SUMMARY OF THE INVENTION

Accordingly it is an object of the present invention to provide a yarn tension controller for maintaining uniform yarn tension for delivery to a downstream yarn processing station.

It is another object of the invention to provide a yarn tension controller which allows to set a desired tension level and tension uniformity downstream from the yarn tension controller.

It is another object of the invention to provide a yarn tension controller which includes means for uniformly and simultaneously setting the yarn tension on a plurality of yarns being processed.

It is another object of the invention to provide a yarn tension controller where each unit can be individually trimmed to fine-adjusted it to suit specific needs in a downstream yarn processing station.

It is another object of the invention to provide a multiple set of yarn tension controllers for which the desired tension level in all yarns can be changed simultaneously to fit a specific need in a downstream yarn processing station.

These and other objects of the present invention are achieved by providing a yarn tension controller by applying a drag force to a whorl around which the yarn is wrapped to achieve a desired tension. If the incoming strand has no tension, the full drag force is applied to the whorl. If the incoming strand has tension, the drag force is proportionally reduced.

It is an object of the invention to achieve the drag force to the whorl by mechanical means.

It is another object of the invention to achieve the drag force to the whorl by electrical means.

It is an object in the preferred embodiments disclosed below to provide a mechanical yarn tension controller, comprising a yarn guiding entrance, a pivoted yarn whorl assembly, a stationary braking means for the whorl, a tensioning pin as a force applying means and a yarn exiting guide. The yarn whorl is by itself freewheeling and the yarn whorl assembly is pivoted at it's bearing extension. This allows the yarn whorl assembly to pivot in plane defined by the direction of the entering yarn and the rotational axis of the yarn whorl. The yarn whorl assembly is pushed away from the entering yarn by a tension pin, which presses the yarn whorl against a stationary brake shoe.

It is another object of the invention to apply a brake to a freely rotating whorl by exerting a force to a brake shoe and then reducing this force through the tension of the incoming yarn strand to achieve a constant out-put tension downstream strand.

It is another object of the invention to achieve a constant out-put tension in the yarn by a yarn tension controller, comprising a yarn guiding entrance, a yarn whorl assembly, an electromagnet which is applying a drag force to the whorl through its eddy-current, a redirection of the incoming up-stream yarn strand and a tension sensing transducer at the point of redirection of the incoming up-stream yarn strand which generates a voltage change at the electromagnet which reduces the magnetic braking force of the whorl correspondingly.

It is another object of the invention to achieve a constant out-put tension in the yarn by a yarn tension controller, comprising a yarn guiding entrance and a yarn whorl assembly and an electro-magnetic braking means for the whorl. The yarn whorl is by itself freewheeling and the yarn whorl assembly is pivoted at it's stationary bearing extension. This allows the yarn whorl assembly to pivot in the plane of the entering yarn and the yarn whorl axis. An electric transducer between the pivotal whorl assembly and the fixed body of the tension controller measures the tension in the incoming yarn strand and reduces the applied tension by the same amount.

It is another object of the invention to achieve a constant out-put tension in the yarn by a yarn tension controller, comprising a yarn guiding entrance and a yarn whorl assembly and an electro-magnetic braking means for the whorl. The yarn whorl is by itself freewheeling and the yarn whorl assembly is mounted onto a flexible support strip which is deflected by the tension in the incoming yarn towards this incoming yarn strand. This flexible support strip is equipped with an electric transducer measuring its deformation and reduces the applied tension by the same amount.

It is an object of the invention to have a mechanical tension controller, where the tension force of the exiting yarn strand is perpendicular to the swinging motion of the whorl assembly so as not to influence the brake.

It is an object of the invention to have an electrical tension controller where the tension force of the exiting yarn strand is perpendicular to the measuring direction of the transducer so as not to influence the measurement of the transducer.

It is an object of the invention to have the tension force of the entering upstream portion of the yarn in opposition direction to the applied braking force of the mechanical yarn tension controller and through this method reducing the preset controller tension, resulting in a constant output tension regardless of tension fluctuations in the upstream yarn strand.

It is another object of this invention to use different geometrical force multipliers to compensate for different coefficient of friction between the brake shoe and the yarn whorl. This geometrical force multiplier can be of various designs as for example the usage of a larger whorl diameter for the brake shoe, then for the yarn, if the coefficient of friction is smaller than one. Other methods of force multiplication can be used to compensate for different friction coefficients as are well known in physics such as the application of a leverage system or applying the force in wedge form.

According to one preferred embodiment of the invention, the friction means comprises a stationary brake shoe inside of the rotating yarn whorl, a pivotal yarn whorl assembly and a force means to engage the brake shoe and the yarn whorl.

According to a preferred embodiment of the invention, the position of the brake shoe can be individually changed to alter the geometrical multiplication factor which compensates for different friction coefficient.

According to another preferred embodiment of the invention, the force means comprises a pressure responsive expandable fluid reservoir.

According to yet another preferred embodiment of the invention, the fluid reservoir comprises a tube and includes pressure adjusting means for adjusting the pressure within the reservoir. Preferably, the fluid comprises air.

According to another preferred embodiment of the invention, tension range adjustment means are provided for adjusting the range of tension applied by the friction means.

According to another preferred embodiment of the invention, an individual fine-tuning of each yarn tension controller is provided to decrease or increase the set tension applied to the individual yarn of a selected tension controller.

According to one preferred embodiment of the invention, the air tube extends to the plurality of yarn tension controllers for simultaneously and uniformly control of the force applied to the yarn at each of the plurality of yarn tension controllers by the tension shoes.

According to one preferred embodiment of the invention, the fluid pressure of all air tubes in a processing system can be automatically raised or lowered, as for example during a speed change of the process.

According to yet another preferred embodiment of the invention, the step of applying a maximum desired pre-set tension to the yarn between the yarn supply station and the yarn processing station comprises applying the tension from a single fluid filled pressure reservoir to each of the yarn tension controllers uniformly and simultaneously.

According to yet another preferred embodiment of the invention the force means to apply pressure to the brake is a spring.

According to yet another preferred embodiment of the invention the force means to apply pressure to the brake is a magnet.

According to yet another preferred embodiment of the invention the force means to apply pressure to the brake is a weight.

According to yet another preferred embodiment of the invention the force means to apply pressure to the brake is fluid pressure.

BRIEF DESCRIPTION OF THE DRAWINGS

Some of the objects of the invention have been set forth above. Other objects and advantages of the invention will appear as the invention proceeds when taken in conjunction with the following drawings, in which:

FIG. **1** is a perspective view of the tension controller according to one embodiment of the invention;
FIG. 1A is a simplified schematic perspective view of the path of the strand from the supply to the take-up according to an embodiment of the invention;
FIG. **2** is a front view of the tension controller viewing it in the direction of the exiting yarn strand;
FIG. **3** is a left side view of the tension controller with the exiting yarn strand leaving to the left;
FIG. **4** is a back view of the tension controller with the yarn strand entering the tension controller from the left;
FIG. **5** is a top view of the tension controller viewing with the yarn strand entering the tension controller from the right and exiting to the top;

FIG. **6** is a front view of the tension controller viewing it in the direction of the exiting yarn with showing the hidden parts in dashed lines;
FIG. **7** is a sectioned front view of the tension controller viewing it in the direction of the exiting yarn;
FIG. **8** shows how the brake shoe is mounted in the brake bracket;
FIG. **9** shows in detail how the brake with its brake bracket and can be rotated around the axis of the arc of the brake block for adjustment of the friction multiplier;
FIG. **10** is a force diagram and demonstrates how the braking force can be adjusted for a different coefficient of friction between the brake shoe and the inside of the yarn whorl.
FIG. **11** is also a force diagram and shows how the upstream tension in the incoming yarn strand reduces the braking tension of the tension compensator;
FIG. **12** is an exploded view of the tension controller with all parts shown. Center lines connect the individual parts to facilitate the understanding of how the parts fit together;
FIG. **13** is a sectioned front view of an electronic tension controller, viewing it in the direction of the exiting yarn with a pressure transducer between the pivotal whorl assembly and the body of the tension controller and an electro-magnet mounted in the whorl;
FIG. **14** is a sectioned front view of an electronic tension controller, viewing it in the direction of the exiting yarn where the whorl assembly is mounted on a spring-leaf with a strain gauge attached to it and an electro-magnet mounted in the whorl;
FIG. **15** is a top view of an electronic tension controller yarn with a solidly mounted whorl and a spring-leaf arm with a strain gauge attached to it to measure the tension in the incoming yarn strand;
FIG. **16** is a top view of an electronic tension controller yarn with a solidly mounted whorl and a pivotal yarn arm which relieves the brake pressure partially.

DESCRIPTION OF THE PREFERRED EMBODIMENT AND BEST MODE

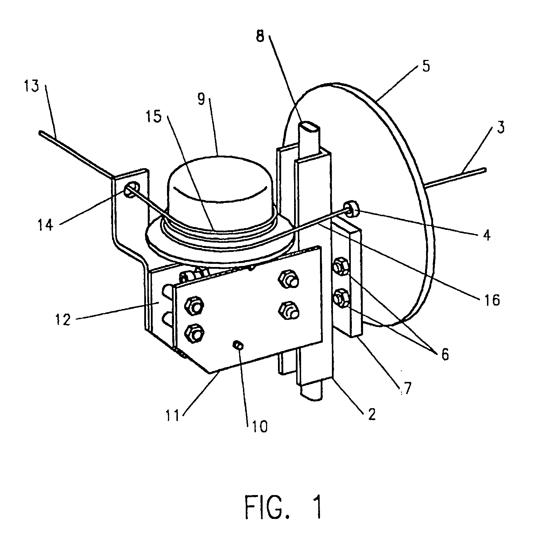

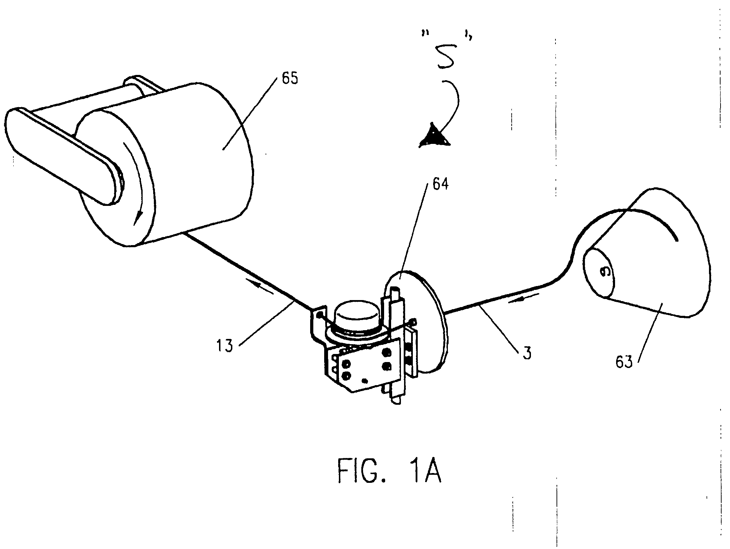

Referring now specifically to the drawings, a tension controller is illustrated in FIG. **1** and broadly illustrated at "T." The tension controller "T" is shown in its environment as a part of a strand tension apparatus, including a strand supply and take-up mechanism in FIG. 1A at "S."

All of the components of the tension controller "T" are mounted on a vertical u-channel **2**. An incoming yarn strand **3** is guided through a guide **4** mounted in a shield disk **5** which in turn is fastened by two screw assemblies **6** onto a mounting angle **7**. The u-channel **2** may be of any desired length and is fastened to a machine frame, creel etc. (not shown). An air tube **8** containing compressed air is located inside the u-channel **2**. A yarn whorl **9** is pivotally mounted through an axle **10** in the axle **10** and front plate **11**. The exiting yarn strand **13** leaves the tension controller "T" through the exit guide **14**. Sufficient yarn wraps **15** are laid around the yarn whorl **9** to assure a slip free contact of the yarn wraps **15** with the yarn whorl **9**.

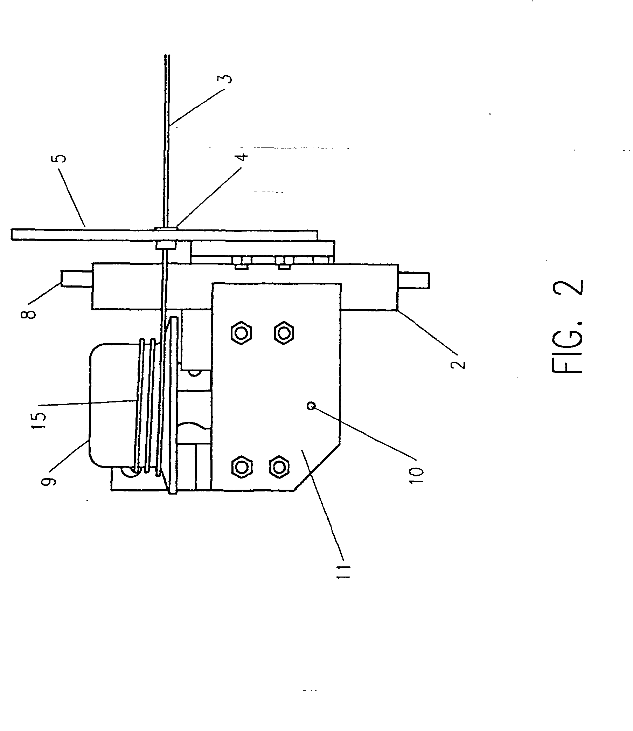

Referring now to FIG. **2**, the same parts are shown in front view.

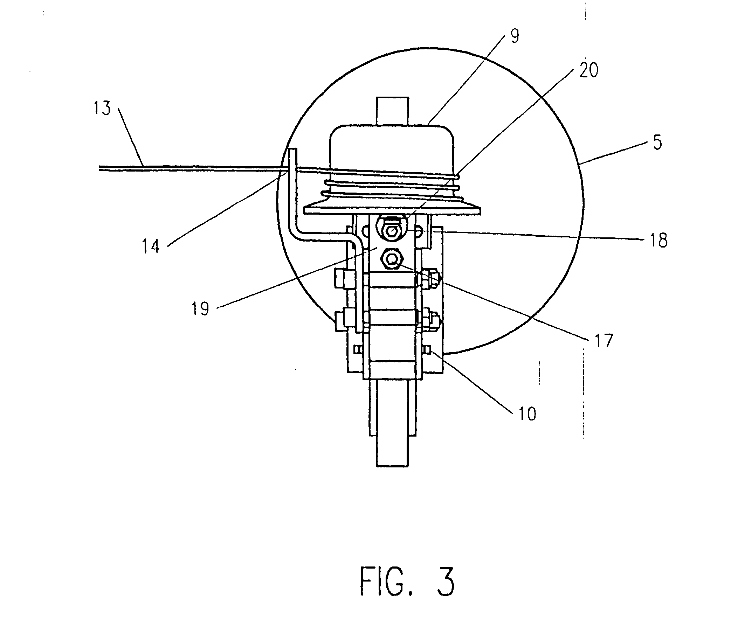

Referring now to FIG. **3**, the left view shows the adjustment screw **17** for the individual tension adjustment. Through the access hole **18** of the stem **19** the brake setting screw **20** can be seen.

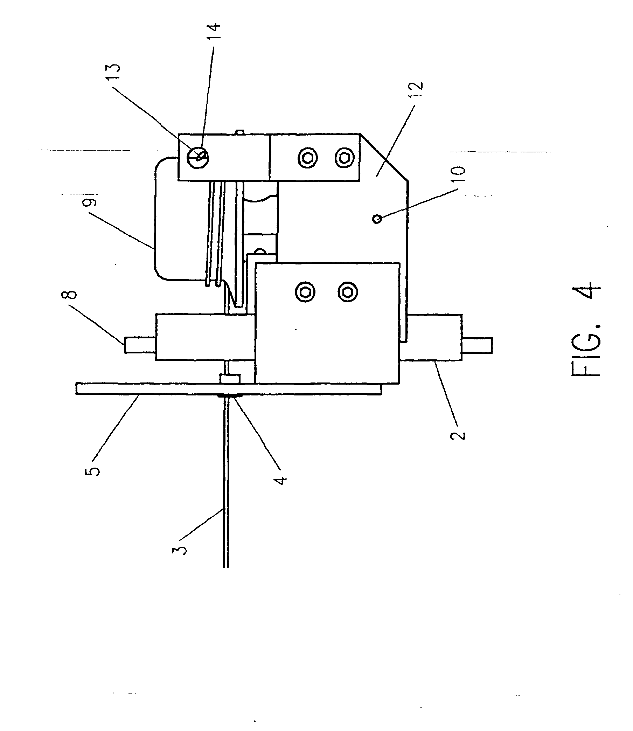

Referring now to FIG. **4**, the tension controller "T" is shown in the back view with the exiting yarn strand **13** guided by the exit guide **14**.

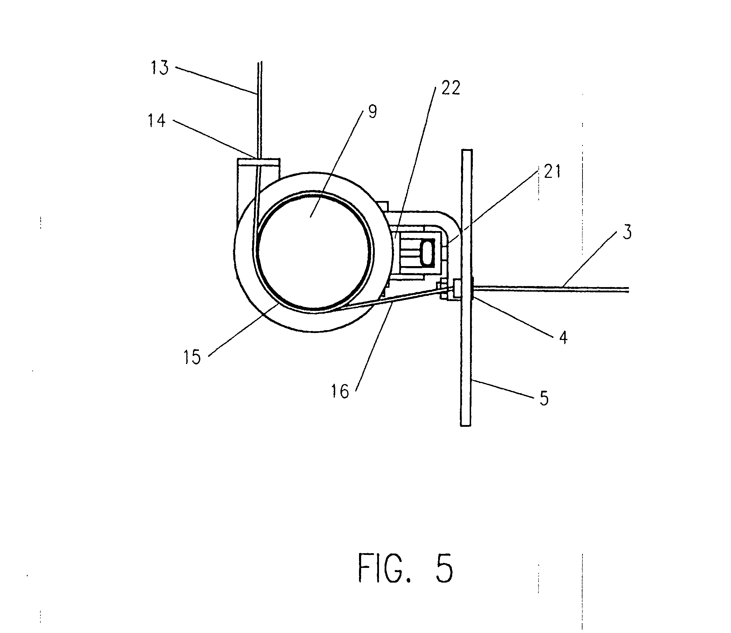

Referring now to FIG. **5**, the incoming yarn strand **3**, yarn strand **16**, yarn wraps **15** and exiting yarn strand **13** are shown from the top. A set screw **21**, threaded into the mounting angle **7** pushes against the u-channel **2** which is held on the opposite side against the brake block **22**.

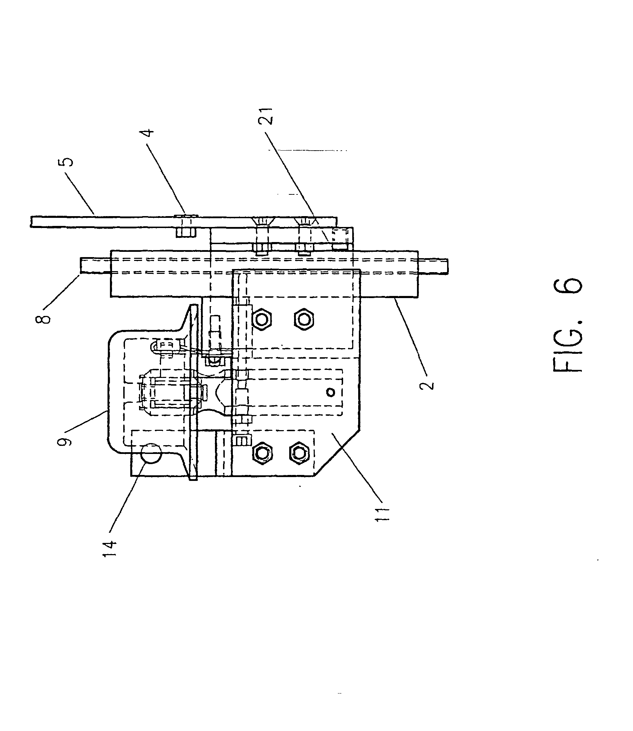

Referring now to FIG. **6**, all hidden parts are shown from the front view.

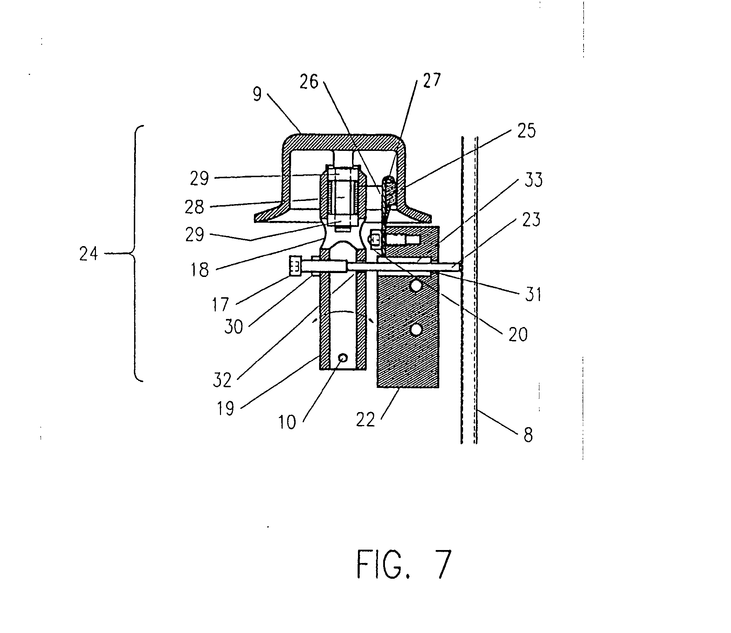

FIG. **7** shows the tension inducing mechanism in a sectional front view. The pressurized air tube **8** pushes the connecting pin **23** toward the left against the adjustment screw **17**. Since the adjustment screw **17** is screwed into the stem **19** this force tries to rotate the whorl assembly **24** counter clockwise around it's axle **10**. A locking nut **30** assures that the setting of the adjustment screw **17** does not change. The whorl assembly **24** is prevented from rotating counter clockwise by the brake shoe **25**. The brake shoe **25** is mounted onto the brake bracket **26** by bracket tabs **27**. The yarn whorl **9** is mounted in the stem **19** by it's whorl axle **28** through two ball bearings **29** and can freely rotate around it's whorl axle **28**. The brake bracket **26** is fastened unto the brake block **22** by a brake setting screw **20**. The connecting pin **23** is loosely held by the block hole **31** in the brake block **22** and on the left in the pin hole **32** of the stem **19**. A relieve hole **33** in the brake block **22** assures that the connecting pin **23** can transmit the tension force unhindered from the air tube **8** to the whorl assembly **24**. The whorl assembly **24** consists of yarn whorl **9**, ball bearing **29**, whorl axle **28**, adjustment screw **17**, stem **19** with the axle **10.**

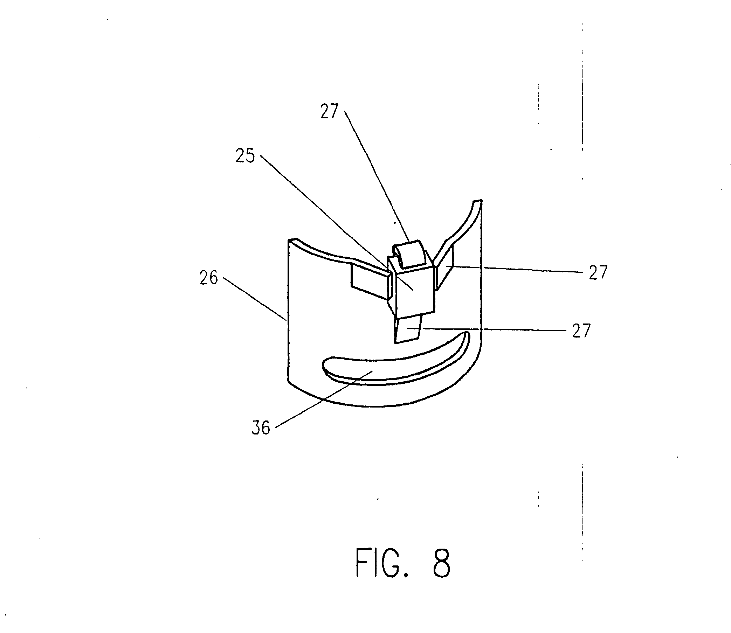

FIG. **8** shows the detail of the brake shoe **25** held through bracket tabs **27** in the brake bracket **26**.

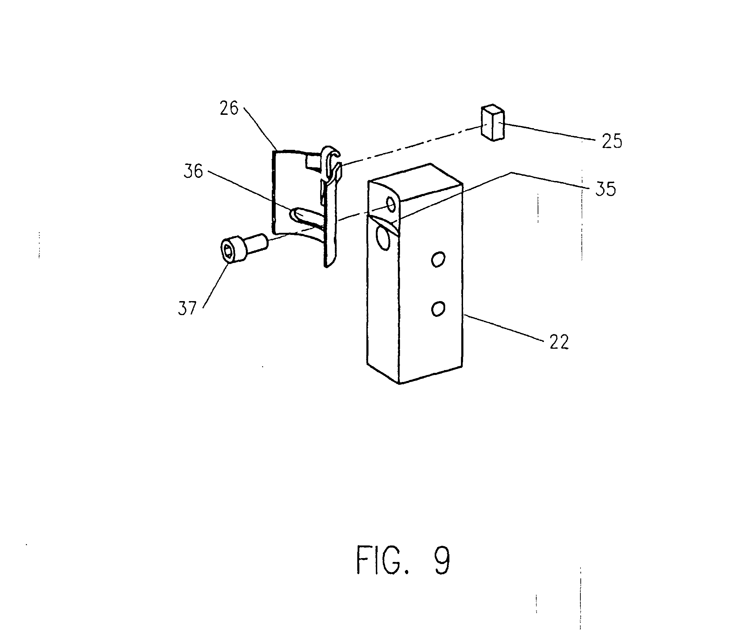

Referring now to FIG. **9**, the mounting of the brake bracket **26** to the brake block **22** is shown in more detail. The brake block **22** is provided with an arc shaped cut-out **35** which has it's center in line with the center of the whorl assembly **24**. The brake bracket **26** is attached to the brake block **22** by a bracket screw **37** through the adjustment slot **36** which allows the brake bracket **26** to be rotated in relation to the brake block **22** for proper adjustment.

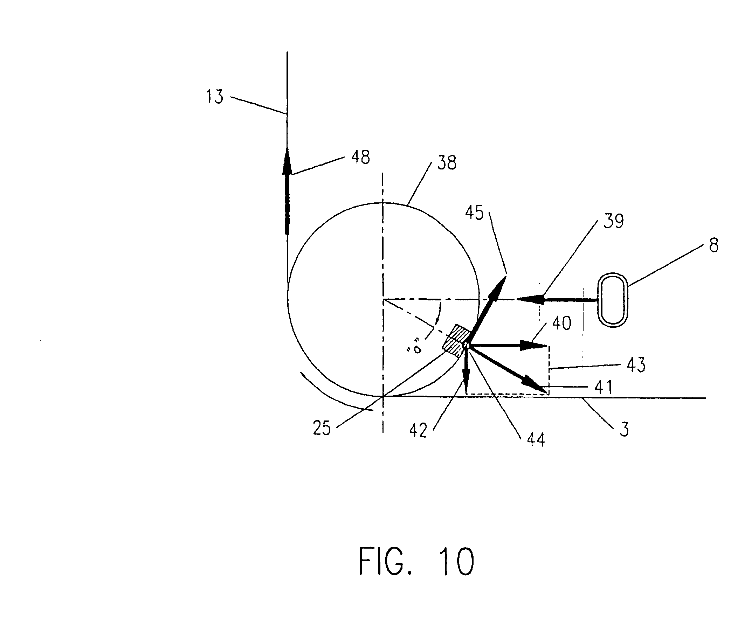

FIG. **10** is a force diagram. The air tube **8** pushes the whorl assembly **24** by it's air pressure in the direction of 9:00 o'clock. The inside surface **38** of the yarn whorl **9** pushes against the fixed brake shoe **25** and through its friction generates the applied tension force for the exiting yarn strand **13**. The forces involved are shown through force vectors. The generating loading force **39** creates at the brake shoe **25** a reaction force **40**. The force parallelogram **43** demonstrates the force multiplication in this system as follows: Since the reaction force **40** is not perpendicular to the brake shoe **25** at contact point **44** the reaction force **40** is broken up into a normal force **41** and a side thrust **42**. The side thrust **42** is counteracted in the axle **10** of the whorl assembly **24** and does not play any function in the tension generation of the tension controller "T". Since it is counteracted in the axle **10** of the whorl assembly **24**. The product of the normal force **41** and the friction coefficient of the brake shoe **25** and the inside surface **38** generates a drag force **45** which is tangential to the inside surface **38** at the contact point **44** of the brake shoe **25**. This drag force **45** is generating the desired yarn tension **48** in the exiting yarn strand **13**. It should be noted that the cosine function of the angle "a" is equal to the friction coefficient between the brake shoe **25** and the inside surface **38**.

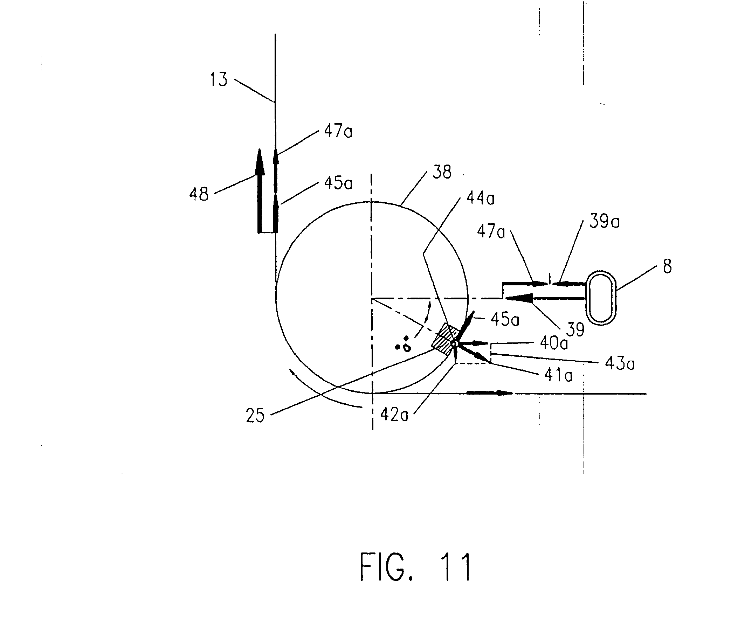

FIG. **11** is also a force diagram and shows the effect of the upstream tension **47a** of the incoming yarn strand **3**. The set loading force **39** is reduced by the upstream tension **47a** resulting in an effective loading force **39a** which is acting on the whorl assembly **24**. The generating loading force **39a** creates at the brake shoe **25** a reaction force **40a**. The force parallelogram **43a** demonstrates the force multiplication in this system as follows: Since the reaction force **40a** is not perpendicular to the brake shoe **25** at contact point **44a** the reaction force **40a** is broken up into a normal force **41a** and a side thrust **42a.** The side thrust **42a** is counteracted in the axle **10** of the whorl assembly **24** and does not play any function in the tension generation of the tension controller "T". The product of the normal force **41a** and the friction coefficient of the brake shoe **25** and the inside surface **38** generates a drag force **45a** which is tangential to the inside surface **38** at the contact point **44** of the brake shoe **25**. The two tension components upstream tension **47a** and drag force **45a** result in a combined yarn tension **48** in the exiting yarn strand **13**.

The tension controller "T" has a constant yarn tension **48** in the exiting yarn strand **13**. The following equation establishes that the exiting yarn strand **13** is controlled in this manner:

Legend:

T1 = Tension in up-stream yarn strand
T2 = Set drag of the tension device
T3 = Drag of the device after reduction by T1

T4 = Tension of down-stream yarn
u1 = Friction coefficient between brake and whorl
"a" = Offset angle of the brake shoe

Calculation for zero up-stream tension T1:
T4 = T2
Calculation of added tension T3:

T3 = T2 - T1 (by definition)

Calculation with up-stream tension T1:

T4 = T1 + T3 = T1 + (T2 - T1)

from this follows:
T4 = T2 (T4 not affected by T1 and constant since T2 is constant)
Calculation of offset angle "a" of the brake shoe:
u1 = cos"a"
and from this:
"a" = arccos(u1)

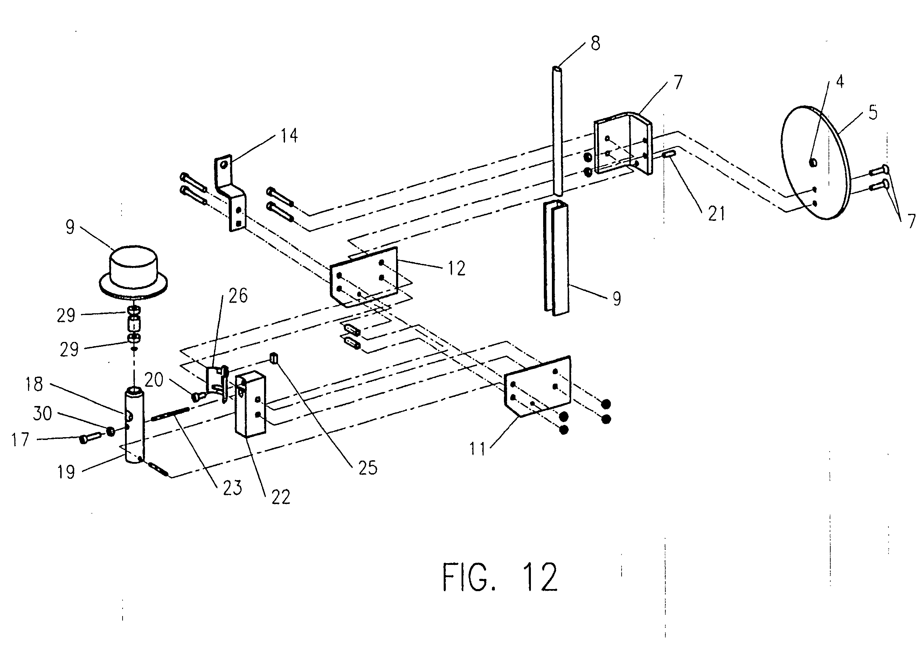

FIG. **12** is an exploded view of the tension controller "T" with all parts shown. Center lines connect the individual parts to facilitate the understanding of how the parts fit together.

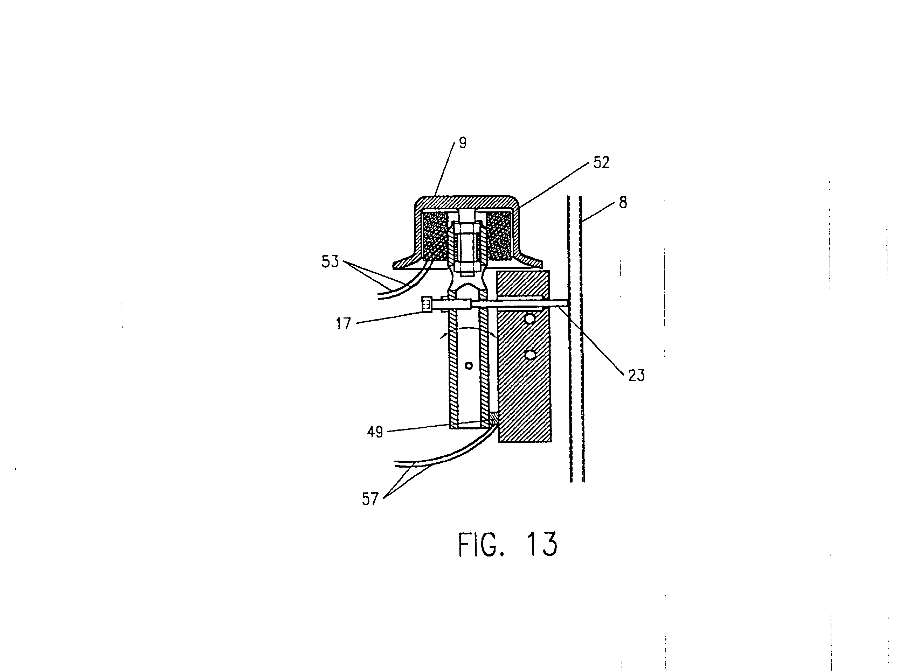

In FIG. **13** a variation of the tension controller "T" is shown with the braking force to the yarn whorl **9** generated by an electro-magnet **52**. The braking force is achieved by applying a voltage through the electrical wires **53** to the electro-magnet **52** and is generated by the effect known as "eddy-current". A pressure transducer **49** is connected by electrical wires **57** to the electro-magnet **52** in series to reduce the voltage to the electrical wires **53** by which means the braking force to the yarn whorl **9** is reduced. If needed, electronic amplification (not shown) is added to the output of the pressure transducer **49** and may be properly matched through a potentiometer (not shown).

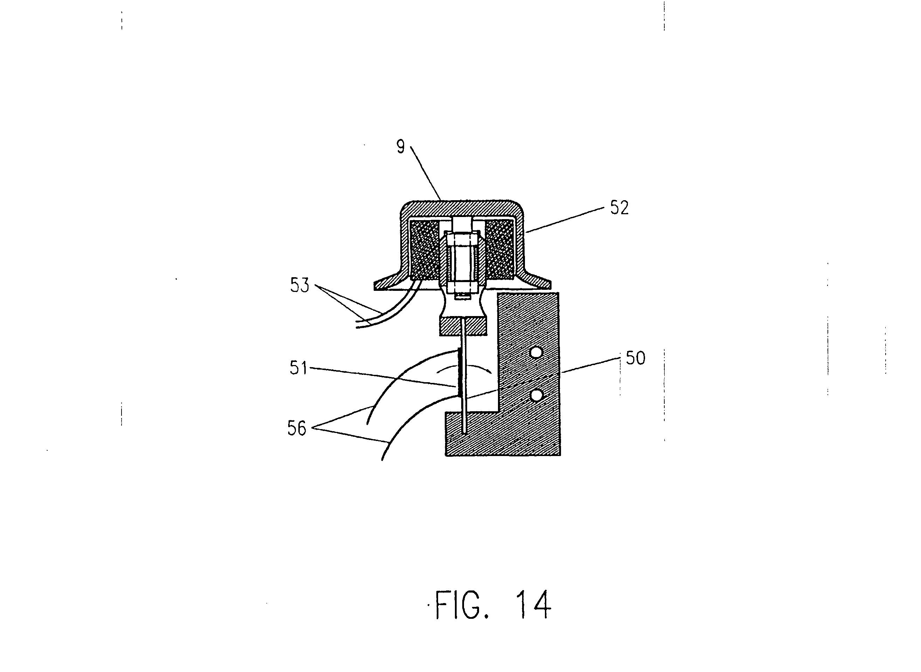

FIG. **14** shows a variation of the method described with FIG. **13**. Instead of the preloading of whorl assembly **24** a spring leaf **50** is employed. The tension of the incoming yarn strand **3** is deflecting the spring leaf **50** and the electrical resistance of an attached strain gauge **51** is changed. This change in resistance is amplified and reduces the voltage to the electro-magnet **52** which in turn reduces the braking force to the yarn whorl **9**. The strain gauge **51** is attached to an amplifier (not shown) by the electrical wires **56**.

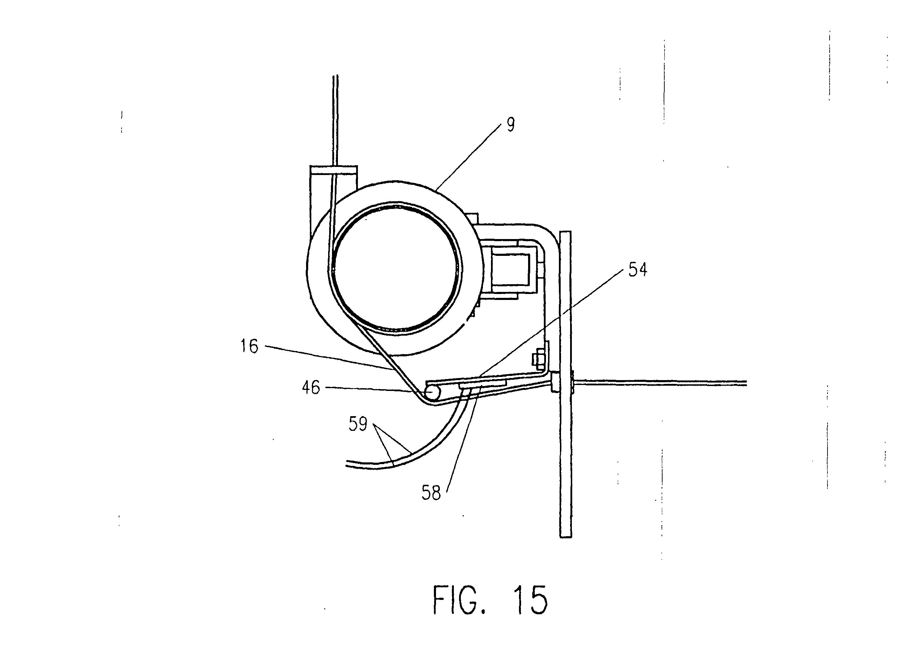

In FIG. **15** the yarn whorl 9 is solidly mounted to the body of the tension controller "T". A spring-leaf arm **54** is deflected by the tension in the yarn strand **16** which is guided around guide **46**. This deflection is also straining the strain gauge **58** and the change in resistance is amplified and reduces the voltage to the electro-magnet **52** inside the yarn whorl **9**, which in turn reduces the braking force to the yarn whorl **9**. The strain gauge **58** is attached to an amplifier (not shown) by electrical wires **59**.

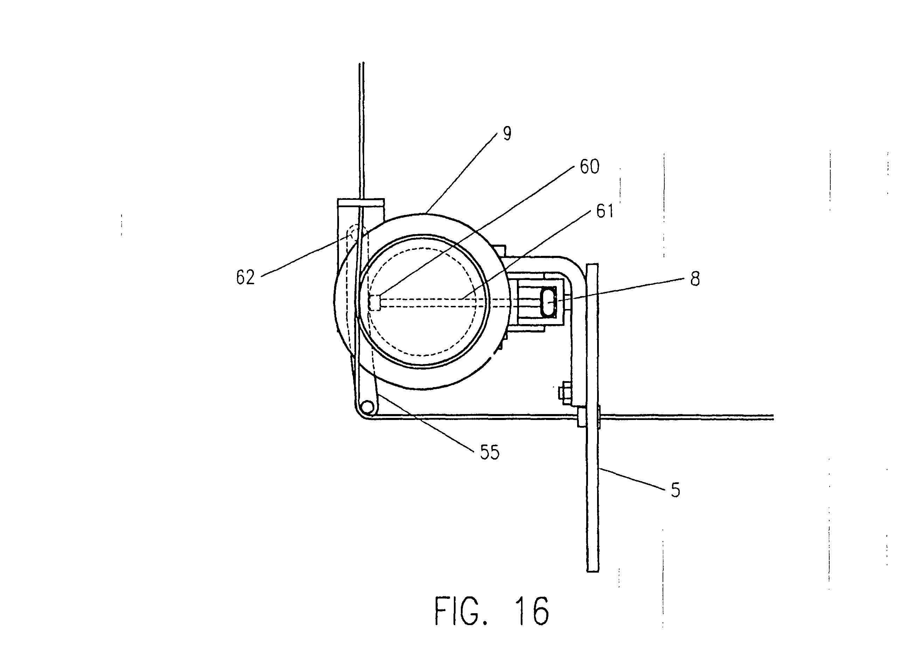

FIG. **16** is a mechanical tension controller "T" with a brake shoe **60** pushing against the inside of a yarn whorl **9** which is solidly mounted to the body of the tension controller "T". The pushing force is determined by the fluid pressure in the air tube **8** and is transmitted to the brake shoe **60** through the connector pin **61**. The yarn arm **55** is pivotal mounted in the body of the tension controller "T" at pivot pin **62** and contacts the brake shoe **60** below the yarn whorl **9**. The yarn arm **55** has a counter-clockwise moment, generated by the tension in the yarn strand **16**. This moment is counteracted at the brake shoe **60** where it reduces the pressure to reduce the braking force at the yarn whorl **9** and thus regulates the tension in the exiting yarn strand.

It will be readily understood by those persons skilled in the art, that the present invention is susceptible of a broad utility and application. Many embodiments and adaptations of the present invention other than those herein described, as well as many variations, modifications, combinations and equivalent arrangements will be apparent from or reasonably suggested by the present invention and the foregoing description thereof, without departing from the substance or scope of the present invention.

**Claims**

1.   A strand tension apparatus, comprising:

(a) a strand delivery mechanism for controllably delivering a moving strand (3) under tension downstream from a strand supply (63);
(b) a strand take-up mechanism (65) positioned downstream from the strand delivery mechanism for pulling the strand from the strand supply;
(c) a tension controller positioned between the strand delivery mechanism and the strand take-up mechanism for adding tension to the moving strand as it moves downstream from the strand delivery mechanism to the strand take-up mechanism, said tension controller including a freewheeling whorl (9) around which the strand is wrapped, the whorl being rotated in use by the strand, the tension controller also including a variable drag force-applying means (8, 17, 25, 52, 60) cooperating with the whorl for adding a predetermined tension to the strand as the strand is delivered downstream from the strand delivery mechanism; and
(d) tension responsive drag-force varying means (8, 17, 25, 52, 60) cooperating with the drag-force applying means and responsive to the tension on the strand being delivered from the strand delivery mechanism for reducing the amount of drag added to the strand by the drag-force applying means by a value resulting in delivery of a strand under uniform tension downstream from the tension controlled to the take-up mechanism.

2. A strand tension apparatus according to claim 1, wherein the tension responsive drag-force adjusting means cooperate with the drag-force applying means responsive to the tension on the strand being delivered from the strand delivery mechanism for reducing the amount of drag added to the strand by the drag-force applying means by a value equal to the tension of the strand upstream from the tension controller to delivery a strand under uniform tension downstream from the tension controller to the take-up mechanism.

3. A strand tension apparatus according to claim 1, wherein the whorl is flexibly mounted and includes a brake for pushing the whorl against a brake shoe (25) to apply a drag force to the whorl for adding tension to said strand.

4. A strand tension apparatus according to claim 1, and including:

(a) a brake shoe flexibly mounted relative to said whorl for applying a braking force against the whorl to apply a drag force to said whorl in order to add tension to said strand; and
(b) a yarn lever around which the incoming yarn is partially deflected for pulling the brake shoe with said yarn lever in an opposite direction as the brake applying force to reduce the applied tension to the yarn strand.

5. A strand tension apparatus according to claim 1, wherein:

(a) the whorl is flexibly mounted, and includes an electric brake for applying a braking force fo said whorl in order to add tension to said strand;
(b) a pressure transducer (49) is provided, against which the strand is pulled in order to create a resistance proportional to a change in tension in the strand upstream of the strand tension controller; and
(c) an electronic amplifier is provided, operatively associated with the transducer for converting the change in resistance in the transducer into a reduction of said holdback force of the whorl.

6. A strand tension apparatus according to claim 1, having:

(a) a yarn lever which is flexibly mounted;
(b) an electric brake for applying a braking force to said whorl in order to add tension to said strand;
(c) a yarn guide on said yarn lever around which the yarn strand is partially deflected;
(d) a transducer (49) for measuring the deflection force of the yarn strand at the yarn guide; and
(e) an electronic amplifier for converting the change in resistance of the transducer caused by the change in pressure into a reduction of said holdback force of the whorl.

7. A strand tension apparatus according to claim 1, wherein the yarn strand downstream of the tension controller is pulled from the whorl in a direction in which the strand tension does not affect the tension on the strand upstream of the tension controller.

8. A strand tension apparatus according to claim 3, and including brake shoe adjustment means (36) for adjusting the position of said brake shoe to thereby change the force multiplication factor to adjust the drag force for the specific coefficient of friction between the brake shoe and the whorl.

9. A strand tension apparatus according to claim 3, and including a spring for applying a braking force to the brake.

10. A strand tension apparatus according to claim 3, and including a magnet for applying a braking force to the brake.

11. A strand tension apparatus according to claim 3, and including an electromagnet (52) for applying a braking force to the brake.

12. A strand tension apparatus according to claim 3, and including a weight for applying a braking force to the brake.

13. A strand tension apparatus according to claim 3, and including a fluid pressure apparatus for applying a fluid pressure braking force to the brake.

14. A strand tension apparatus according to claim 13, and including a fluid pressure adjusting apparatus (8) for varying the fluid pressure applied to the brake.

15. A strand tension apparatus according to claim 13, and including pressure means for applying a like fluid pressure simultaneously to a plurality of tension controllers.

16. A strand tension apparatus according to claim 11, and including pressure means for applying a like electromagnetic force simultaneously to a plurality of tension controllers.

17. A strand tension apparatus according to claim 15 or 16, and including a fine-scale force-adjusting device on each of the plurality of tension controllers for adjusting the braking force independent of each of the other plurality of tension controllers.

18. A strand tension apparatus according to claim 17, wherein said fine-scale adjusting device is adjustable during operation of the strand tension apparatus.

19. A method of controlling strand tension in a moving stand, comprising the steps of:

(a) feeding the strand downstream to a freewheeling whorl of a tension controller, with which whorl the strand is engaged;
(b) adding a desired drag force to the whorl of the tension controller to add tension to the strand;
(c) detecting the tension in the strand moving downstream to the tension controller; and
(d) reducing the drag force on the whorl in response to the tension detected in the strand moving downstream to the tension controller by a value sufficient to deliver the strand under uniform tension downstream from the tension controller to a downstream take-up mechanism.

20. A method of controlling strand tension according to claim 19, where the reduction of said drag force results in a reduction of said added tension equal to the tension of the incoming strand.

21. A method of controlling strand tension according to claim 19 or 20, wherein the tension is applied by applying a drag force to the whorl and by transferring the drag force of the whorl into tension in the strand which is wrapped with sufficient wrapping around the whorl to prevent slippage.

22. A method of controlling strand tension according to claim 21, and including the step of providing a plurality of tension control stations for simultaneous control of a plurality of strands.

**Patentansprüche**

1. Fadenspannungsregelvorrichtung, welche aufweist:

(a) einen Fadenabgabemechanismus zur regelbaren Abgabe eines laufenden Fadens (3) unter Spannung stromabwärts von einer Fadenzufuhr (63);
(b) einen Fadenaufnahmemechanismus (65), der stromabwärts vom Fadenabgabemechanismus angeordnet ist, um den Faden von der Fadenzufuhr abzuziehen;
(c) einen Fadenspannungsregler, der zwischen dem Fadenabgabemechanismus und dem Fadenaufnahmemechanismus angeordnet ist, um dem laufenden Faden Spannung hinzuzufügen, während er vom Fadenabgabemechanismus stromabwärts zum Fadenaufnahmemechanismus läuft, wobei der Spannungsregler einen freilaufenden Wirtel aufweist, um welchen der Faden gewunden ist und der im Betrieb durch den Faden gedreht wird, und der Spannungsregler auch ein Mittel zum Aufbringen einer veränderlichen Rückhaltekraft (8, 17, 25, 52, 60) aufweist, das mit dem Wirtel zusammenwirkt, um dem Faden, während er stromabwärts vom Fadenabgabemechanismus abgegeben wird, eine vorbestimmte Spannung hinzuzufügen; und
(d) ein auf Spannung ansprechendes, die Bremskraft veränderndes Mittel (8, 17, 25, 52, 60), das mit dem die Rückhaltekraft aufbringenden Mittel zusammenwirkt und entsprechend der Spannung am vom Fadenabgabemechanismus abgegebenen Faden die Größe der Rückhaltekraft, welche dem Faden durch das die Rückhaltekraft aufbringende Mittel zugefügt wird, um einen solchen Wert herabsetzt, daß ein Faden unter gleichmäßiger Spannung stromabwärts von der gesteuerten Spannung zum Aufnahmemechanismus abgegeben wird.

2. Fadenspannungsregelvorrichtung nach Anspruch 1, worin das auf die Spannung ansprechende die Bremskraft einstellende Mittel mit dem die Rückhaltekraft aufbringenden Mittel zusammenwirkt, welches auf die Spannung anspricht, die an dem vom Fadenabgabemechanismus abgegebenen Faden herrscht, um die Höhe der dem Faden durch das die Rückhaltekraft aufbringende Mittel zugefügten Rückhaltekraft um einen Wert gleich der Spannung des Fadens stromaufwärts vom Spannungsregler zu verringern, um einen Faden unter gleichmäßiger Spannung stromabwärts vom Spannungsregler zum Aufnahmemechanismus abzugeben.

3. Fadenspannungsregelvorrichtung nach Anspruch 1, worin der Wirtel biegsam montiert ist und eine Bremse aufweist, um den Wirtel gegen einen Bremsschuh (25) zu drükken, um auf den Wirtel eine Rückhaltekraft auszuüben, um dem Faden Spannung hinzuzufügen.

4. Fadenspannungsregelvorrichtung nach Anspruch 1, welche weiter aufweist:

(a) einen relativ zum Wirtel biegsam montierten Bremsschuh zum Aufbringen einer auf den Wirtel wirkenden Bremskraft, um auf den Wirtel eine Rückhaltekraft auszuüben, um dem Faden zusätzliche Spannung zu verleihen; und
(b) einen Fadenhebel, um welchen der zulaufende Faden teilweise umgelenkt wird, um den Bremsschuh mit dem Fadenhebel in eine entgegengesetzte Richtung zur die Bremse andrückenden Kraft zu ziehen, um die auf den Faden aufgebrachte Spannung zu verringern.

5. Fadenspannungsregelvorrichtung nach Anspruch 1, worin

(a) der Wirtel biegsam montiert ist und eine elektrische Bremse aufweist, um auf den Wirtel eine Bremskraft auszuüben, um dem Faden Spannung hinzuzufügen;
(b) ein Druckwandler (49) vorgesehen ist, gegen den der Faden gezogen wird, um einen Widerstand proportional zu einer Veränderung in der Spannung im Faden stromaufwärts vom Fadenspannungsregler zu erzeugen; und
(c) ein elektronischer Verstärker vorgesehen ist, der in Wirkverbindung mit einem Wandler steht, um die Veränderung im Widerstand im Wandler in eine Verringerung der Rückhaltekraft des Wirtels umzuwandeln.

6. Fadenspannungsregelvorrichtung nach Anspruch 1 mit

(a) einem Fadenhebel, der biegsam montiert ist;
(b) einer elektrischen Bremse zum Aufbringen einer Bremskraft auf den Wirtel, um dem Faden Spannung hinzuzufügen;
(c) einem Fadenführer an dem Fadenhebel, um welchen der Fadenstrang teilweise abgelenkt wird;
(d) einem Wandler (49) zum Messen der Ablenkungskraft des Fadenstranges an der Fadenführung; und
(e) einem elektronischen Verstärker zum Umwandeln der Veränderung des Widerstands des Wandlers, der durch die Druckveränderung verursacht ist, in eine Verringerung der Rückhaltekraft des Wirtels.

7. Fadenspannungsregelvorrichtung nach Anspruch 1, worin der Fadenstrang stromabwärts vom Spannungsregler von dem Wirtel in einer Richtung abgezogen wird, in welcher die Fadenspannung die Spannung am Faden stromaufwärts vom Spannungsregler nicht beeinflußt.

8. Fadenspannungsregelvorrichtung nach Anspruch 3, welche weiter ein Bremsschuheinstellmittel (36) aufweist, um die Stellung des Bremsschuhs einzustellen und **dadurch** den Kraftverstärkungsfaktor zu verändern, um die Rückhaltekraft für den spezifischen Reibungskoeffizienten zwischen dem Bremsschuh und dem Wirtel einzustellen.

9. Fadenspannungsregelvorrichtung nach Anspruch 3 mit einer Feder zum Aufbringen einer Bremskraft auf die Bremse.

10. Fadenspannungsregelvorrichtung nach Anspruch 3 mit einem Magneten zum Aufbringen der Bremskraft, auf die Bremse.

11. Fadenspannungsregelvorrichtung nach Anspruch 3 mit einem Elektromagneten (52) zum Aufbringen einer Bremskraft auf die Bremse.

12. Fadenspannungsregelvorrichtung nach Anspruch 3 mit einem Gewicht zum Aufbringen einer Bremskraft auf die Bremse.

13. Fadenspannungsregelvorrichtung nach Anspruch 3 mit einer Fluiddruckvorrichtung zum Aufbringen einer Fluiddruck-Bremskraft auf die Bremse.

14. Fadenspannungsregelvorrichtung nach Anspruch 13, welche außerdem eine Fluiddruck-Einstellvorrichtung (8) aufweist, um den auf die Bremse ausgeübten Fluiddruck zu verändern.

15. Fadenspannungsregelvorrichtung nach Anspruch 13, welche außerdem Druckmittel aufweist, um einen gleichen Fluiddruck gleichzeitig auf eine Mehrzahl von Spannungsreglern auszuüben.

16. Fadenspannungsregelvorrichtung nach Anspruch 11, welche außerdem Druckmittel aufweist, um eine gleiche elektromagnetische Kraft gleichzeitig auf eine Mehrzahl von Spannungsreglern auszuüben.

17. Fadenspannungsregelvorrichtung nach Anspruch 15 oder 16, welche außerdem an jeder der Mehrzahl der Spannungsregler eine feinabgestufte Krafteinstellvorrichtung aufweist, um die Bremskraft unabhängig von jedem der anderen der Mehrheit der Spannungsregler einzustellen.

18. Fadenspannungsregelvorrichtung nach Anspruch 17, worin die Feinabstimmungseinstellvorrichtung während des Betriebs der Fadenspannungsregelvorrichtung einstellbar ist.

19. Verfahren zur Steuerung der Fadenspannung in einem laufenden Faden mit folgenden Verfahrensschritten:

(a) Zuführen des Fadens stromabwärts zu einem freilaufenden Wirtel eines Fadenspannungsreglers, wobei der Faden mit diesem Wirtel in Eingriff ist;

(b) Zufügen einer gewünschten Rückhaltekraft zum Wirtel des Fadenspannungsreglers, um dem Faden zusätzliche Spannung zu geben;

(c) Detektieren der Spannung in dem stromabwärts zum Spannungsregler laufenden Faden; und

(d) Verringern der Rückhaltekraft an dem Wirtel entsprechend der in dem stromabwärts zum Spannungsregler laufenden Faden detektierten Spannung um einen Wert, der ausreicht, um den Faden unter gleichmäßiger Spannung stromabwärts vom Spannungsregler zu einem Stromabwärts-Aufnahmemechanismus abzugeben.

20. Verfahren zum Steuern der Fadenspannung nach Anspruch 19, wobei die Verringerung der Rückhaltekraft zu einer Verringerung der zugefügten Spannung führt, die gleich der Spannung des zulaufenden Fadens ist.

21. Verfahren zur Steuerung der Fadenspannung nach Anspruch 19 oder 20, wobei die Spannung aufgebracht wird, indem eine Rückhaltekraft auf den Wirtel ausgeübt wird und die Rückhaltekraft des Wirtels in Spannung in dem Faden übertragen wird, der mit genügend Windungen um den Wirtel gewunden ist, um Schlupf zu verhindern.

22. Verfahren zum Steuern der Fadenspannung nach Anspruch 21 mit dem Schritt, daß eine Mehrzahl von Spannungsregelstationen für die gleichzeitige Steuerung einer Mehrzahl von Fäden vorgesehen ist.

## Revendications

1. Appareil de tension de fil, comprenant :

(a) un mécanisme de distribution de fil destiné à distribuer de façon contrôlable un fil mobile (3) sous tension, en aval du dispositif d'alimentation en fil (63) ;

(b) un mécanisme de prise de fil (65) positionné en aval par rapport au mécanisme de distribution de fil destiné à tirer le fil provenant du dispositif d'alimentation en fil ;

(c) un contrôleur de tension positionné entre le mécanisme de distribution de fil et le mécanisme de prise de fil destiné à ajouter de la tension sur le fil mobile lorsqu'il se déplace en aval du mécanisme de distribution de fil vers le mécanisme de prise de fil, le contrôleur de tension comprenant une spirale en roue libre (9) autour de laquelle le fil est enroulé, la spirale étant tournée lors de l'utilisation par le fil, le contrôleur de tension comprenant également des moyens d'application de force de traînée variable (8, 17, 25, 52, 60) coopérant avec la spirale afin d'ajouter une tension prédéterminée sur le fil lorsque le fil est acheminé en aval du mécanisme de distribution de fil ; et

(d) des moyens de variation de force de traînée sensibles à la tension (8, 17, 25, 52, 60) coopérant avec les moyens d'application de force de traînée et sensibles à la tension appliquée sur le fil distribué par le mécanisme de distribution de fil afin de réduire la quantité de traînée ajoutée au fil par les moyens d'application de force de traînée d'une valeur résultant en une distribution d'un fil sous tension constante en aval du contrôleur de tension vers le mécanisme de prise.

2. Appareil de tension de fil selon la revendication 1, dans lequel
les moyens d'ajustement de force de traînée sensibles à la tension coopèrent avec les moyens d'application de force de traînée sensibles à la tension appliquée sur le fil distribué par le mécanisme de distribution de fil afin de réduire la quantité de traînée ajoutée au fil par les moyens d'application de force de traînée d'une valeur égale à la tension du fil en amont par rapport au contrôleur de tension pour distribuer un fil sous tension constante en aval du contrôleur de tension vers le mécanisme de prise.

3. Appareil de tension de fil selon la revendication 1, dans lequel
la spirale est montée de manière flexible et comprend un frein destiné à pousser la spirale contre un patin de frein (25) afin d'appliquer une force de traînée à la spirale pour ajouter de la tension sur ledit fil.

4. Appareil de tension de fil selon la revendication 1, comprenant :

(a) un patin de frein monté de manière flexible par rapport à ladite spirale afin d'appliquer une force de freinage contre la spirale pour appliquer une force de traînée à la spirale afin d'ajouter de la tension sur le fil ; et

(b) un levier de fil autour duquel le fil arrivant est partiellement dévié pour tirer le patin de frein avec le levier de fil dans une direction opposée comme un frein appliquant une force afin de réduire la tension du fil.

5. Appareil de tension de fil selon la revendication 1,

dans lequel :

(a) la spirale est montée de manière flexible, et comprend un frein électrique destiné à appliquer une force de freinage sur ladite spirale afin d'ajouter de la tension sur ledit fil ;

(b) un capteur de pression (49) est utilisé, contre lequel le fil est tiré afin de créer une résistance proportionnelle à un changement de tension du fil en amont du contrôleur de tension de fil ; et

(c) un amplificateur électronique est utilisé, associé de façon fonctionnelle au capteur pour convertir le changement de résistance dans le capteur en une réduction de la force de retenue de la spirale.

6. Appareil de tension de fil selon la revendication 1, comprenant :

(a) un levier de fil qui est monté de manière flexible ;

(b) un frein électrique destiné à appliquer une force de freinage sur ladite spirale afin d'ajouter de la tension sur ledit fil;

(c) un guide de fil situé sur le levier de fil autour duquel le fil est partiellement dévié ;

(d) un capteur (49) destiné à mesurer la force de déviation du fil au niveau du guide de fil ; et

(e) un amplificateur électronique destiné à convertir le changement de résistance du capteur causé par le changement de pression en une réduction de la force de retenue de la spirale.

7. Appareil de tension de fil selon la revendication 1, dans lequel le fil en aval du contrôleur de tension est tiré de la spirale dans une direction dans laquelle la tension du fil n'affecte pas la tension du fil situé en amont du contrôleur de tension.

8. Appareil de tension de fil selon la revendication 3, comprenant un moyen d'ajustement du patin de frein (36) destiné à ajuster la position dudit patin de frein pour changer le facteur de multiplication de force afin d'ajuster la force de traînée pour le coefficient spécifique de frottement entre le patin de frein et la spirale.

9. Appareil de tension de fil selon la revendication 3, comprenant un ressort destiné à appliquer une force de freinage sur le frein.

10. Appareil de tension de fil selon la revendication 3, comprenant un aimant destiné à appliquer une force de freinage sur le frein.

11. Appareil de tension de fil selon la revendication 3, comprenant un électroaimant (52) destiné à appliquer une force de freinage sur le frein.

12. Appareil de tension de fil selon la revendication 3, comprenant un poids destiné à appliquer une force de freinage sur le frein.

13. Appareil de tension de fil selon la revendication 3, comprenant un appareil à pression de fluide destiné à appliquer une force de freinage à pression de fluide sur le frein.

14. Appareil de tension de fil selon la revendication 13, comprenant un appareil d'ajustement de pression de fluide (8) destiné à faire varier la pression de fluide appliquée sur le frein.

15. Appareil de tension de fil selon la revendication 13, comprenant un moyen pression de fluide destiné à appliquer simultanément une même pression sur une pluralité de contrôleurs de tension.

16. Appareil de tension de fil selon la revendication 11, comprenant un moyen pression destiné à appliquer simultanément une même force électromagnétique sur une pluralité de contrôleurs de tension.

17. Appareil de tension de fil selon la revendication 15 ou 16, comprenant un dispositif d'ajustement de force à petite échelle sur chaque contrôleur de tension de la pluralité de contrôleurs de tension, pour ajuster la force de freinage indépendante de chaque contrôleur de tension de l'autre pluralité de contrôleurs de tension.

18. Appareil de tension de fil selon la revendication 17, dans lequel ledit dispositif d'ajustement à petite échelle est ajustable pendant le fonctionnement de l'appareil de tension de fil.

19. Procédé de contrôle de tension de fil sur un fil mobile, comprenant les étapes consistant à :

(a) faire descendre le fil en aval vers une spirale en roue libre d'un contrôleur de tension, avec laquelle spirale le fil est engagé ;
(b) ajouter une force de traînée souhaitée à la spirale du contrôleur de tension afin d'ajouter de la tension sur le fil ;
(c) détecter la tension sur le fil qui descend en aval vers le contrôleur de tension ; et
(d) réduire la force de traînée sur la spirale en réponse à la tension détectée sur le fil qui descend en aval vers le contrôleur de tension d'une valeur suffisante pour distribuer le fil sous tension constante en aval du contrôleur de tension vers un mécanisme de prise situé en aval.

20. Procédé de contrôle de tension de fil selon la revendication 19,
   selon lequel
   la réduction de ladite force de traînée résulte en une réduction de ladite tension ajoutée égale à la tension de fil arrivant.

21. Procédé de contrôle de tension de fil selon la revendication 19 ou 20,
   selon lequel
   la tension est appliquée en appliquant une force de traînée sur la spirale et en reportant la force de traînée de la spirale sur la tension du fil qui est enroulé avec un enroulement suffisant autour de la spirale pour éviter tout glissement.

22. Procédé de contrôle de tension de fil selon la revendication 21, comprenant
   l'étape consistant à utiliser une pluralité de stations de contrôle de tension pour le contrôle simultané d'une pluralité de fils.

FIG. 1

FIG. 1A

EP 1 105 335 B1

FIG. 2

FIG. 3

EP 1 105 335 B1

FIG. 4

EP 1 105 335 B1

FIG. 5

FIG. 6

FIG. 7

FIG. 8

FIG. 9

FIG. 10

FIG. 11

EP 1 105 335 B1

FIG. 12

FIG. 13

EP 1 105 335 B1

FIG. 14

FIG. 15

FIG. 16